# EUROPEAN PATENT SPECIFICATION

(11) **EP 4 043 061 B1**
(45) Date of publication and mention of the grant of the patent: **08.07.2026**
(21) Application number: 20873954.0
(22) Date of filing: 14.09.2020
(51) Int. Cl.: A61M 37/00

(54) **MICRONEEDLE SHEET AND APPLICATOR**
MIKRONADELBLATT UND APPLIKATOR
FEUILLE DE MICRO-AIGUILLE ET APPLICATEUR

(30) Priority: 08.10.2019 JP 2019185066
(43) Date of publication of application: 17.08.2022
(73) Proprietor: Hisamitsu Pharmaceutical Co., Inc., Tosu-shi, Saga 841-0017 (JP)
(72) Inventor: YAMAMOTO, Naoki, Tsukuba-shi, Ibaraki 305-0856 (JP); NISHIMURA, Shinpei, Tsukuba-shi, Ibaraki 305-0856 (JP)
(74) Representative: Grünecker Patent- und Rechtsanwälte PartG mbB
(86) International application number: PCT/JP2020/034685
(87) International publication number: WO 2021/070568

(56) References cited:
- EP-A1- 3 391 936
- WO-A1-2015/033959
- WO-A1-2016/088886
- WO-A1-2016/088886
- WO-A1-2017/104491
- WO-A1-2018/116986
- CN-A- 104 379 209
- JP-A- 2006 345 984

## Description

### Technical Field

One aspect of the present disclosure relates to a microneedle sheet and an applicator.

### Background Art

Microneedle sheets used for administering an active ingredient through skin are known. For example, Patent Literature 1 describes a microneedle sheet including a plurality of microneedles formed on a sheet and extending substantially along a main surface of the sheet. Regarding this microneedle sheet, the sheet is bent, whereby the microneedles are raised from the main surface and the raised microneedles are inserted into skin. Further relevant prior art is described in EP 3 391 936 A1, WO 2016/088886 A1, CN 104 379 209 A and WO 2018/116986 A1. Said documents disclose microneedles piercing the skin.

### Citation List

### Patent Literature

[Patent Literature 1] WO 2013/187392

### Summary of Invention

### Technical Problem

It is desired to obtain safety of a microneedle when being handled.

### Solution to Problem

In particular the present invention proposes a microneedle sheet having the features defined in claim 1. Further it is provided an applicator having the features defined in claim 6. Further preferred embodiments are defined in the dependent claims.

### Advantageous Effects of Invention

According to the aspect of the present disclosure, the safety of the microneedle when being handled can be obtained.

### Brief Description of Drawings

FIG. 1 is a plan view illustrating one example of a microneedle sheet according to an embodiment.
FIG. 2 is a diagram illustrating two types of states of the microneedle sheet according to the embodiment.
FIG. 3 is a diagram illustrating one example of a method for using the microneedle sheet according to the embodiment.
FIG. 4 is a diagram illustrating one example of rising of microneedles.
FIG. 5 is a photograph illustrating a result of an in-vitro test for the microneedle sheet according to the embodiment.
FIG. 6 is a perspective view of one example of an applicator according to the embodiment when viewed from below.
FIG. 7 is a perspective view of the example of the applicator according to the embodiment when viewed from above.
FIG. 8 is a diagram illustrating one example of a method for using the applicator according to the embodiment.

### Description of Embodiments

An embodiment of the present disclosure will now be described in detail with reference to the attached drawings. In the description of the drawings, like or equivalent elements are designated by like reference signs, and duplicated explanation is omitted.

### [Structure of Microneedle Sheet]

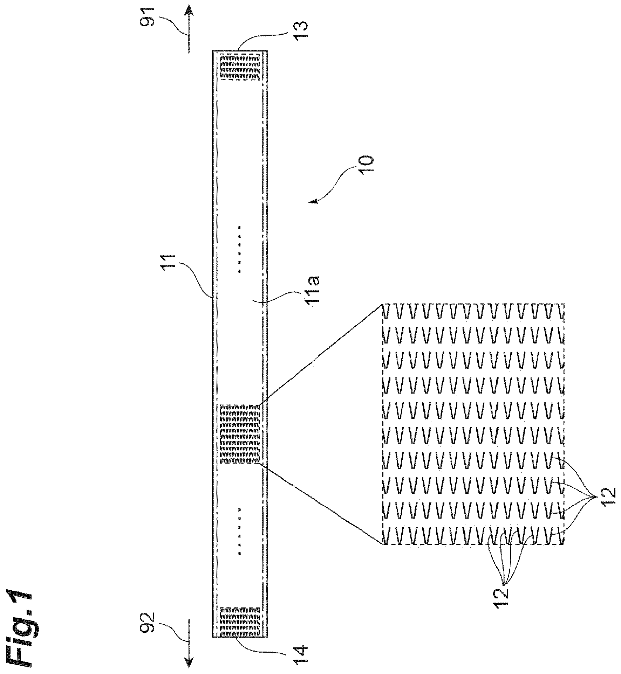

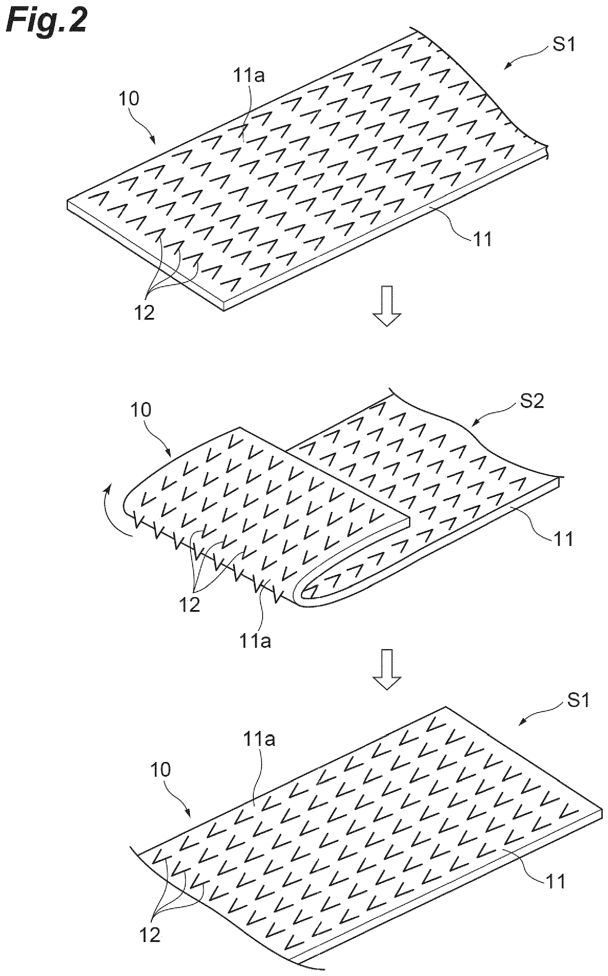

Referring to FIG. 1 and FIG. 2, the following describes a structure of a microneedle sheet 10 according to the embodiment. FIG. 1 is a plan view illustrating one example of the microneedle sheet 10. FIG. 2 is a diagram illustrating two types of states of the microneedle sheet 10.

The microneedle sheet 10 is a tool for transdermally administering any active ingredient (e.g., a pharmaceutical substance) into a living body. In the example of FIG. 1, the microneedle sheet 10 has a belt (elongated rectangular) shape. The microneedle sheet 10 includes a thin plate-like sheet body 11 and at least one microneedle 12 formed on the sheet body 11. In the present embodiment, the direction along the long side of the microneedle sheet 10 is called the longitudinal direction of the microneedle sheet 10 (or of the sheet body 11), and the direction along the short side of the microneedle sheet 10 (direction orthogonal to the longitudinal direction) is called the width direction of the microneedle sheet 10 (or of the sheet body 11). The direction orthogonal to both the longitudinal direction and the width direction is called the thickness direction of the microneedle sheet 10 (or of the sheet body 11). In the present embodiment, both ends of the sheet body 11 in the longitudinal direction are called a first end 13 and a second end 14, a side closer to the first end 13 is called a first side 91, and a side closer to the second end 14 is called a second side 92. The second side 92 is opposite to the first side 91. It can also be said that the longitudinal direction of the sheet body 11 is a direction extending from the first end 13 (first side 91) to the second end 14 (second side 92). The sheet body 11 has a main surface 11a extending from the first end 13 (first side 91) to the second end 14 (second side 92).

In one example, the microneedle sheet 10 includes a plurality of the microneedles 12. The thickness (length along the thickness direction) of each microneedle 12 is the same as the thickness of the sheet body 11. The microneedles 12 are formed such that two or more microneedles 12 are aligned in one row in each of the longitudinal direction and the width direction of the sheet body 11. Each microneedle 12 is formed such that a base thereof is positioned on the first side 91 of the sheet body 11 and a tip thereof is oriented toward the second side 92 of the sheet body 11. In other words, side surfaces of the microneedle 12 extending from the base to the tip of the microneedle 12 are parallel or substantially parallel to the main surface 11a. The orientation of some microneedles 12 may be different from the orientation of the other microneedles 12.

In the present disclosure, a direction in which the tip of each microneedle 12 is oriented, that is, a direction in which the first side and the second side are connected is also called a first direction of the microneedle sheet 10 (or of the sheet body 11). A direction orthogonal to the first direction on the main surface 11a is also called a second direction of the microneedle sheet 10 (or the sheet body 11). In the present embodiment, the first direction is the longitudinal direction, and the second direction is the width direction.

At the time when the microneedle sheet 10 is provided (i.e., at the time before use), the microneedle sheet 10 is straight without being bent. In the present disclosure, this state is also called "first state". When the microneedle sheet 10 is used, the microneedle sheet 10 is brought into a state, in which at least part of the sheet body 11 is bent such that a curved surface is formed in a direction intersecting the orientation of the tip of each microneedle 12. In the present disclosure, this state is also called "second state". For example, the sheet body 11 is bent such that a curved surface is formed along a direction orthogonal to or substantially orthogonal to the orientation of the tip of the microneedle 12. As illustrated in FIG. 2, the microneedle sheet 10 can change from the first state S1 into the second state S2, and also can return from second state S2 to the first state S1. In the second state S2 illustrated in FIG. 2, the sheet body 11 is bent such that a curved surface is formed in a direction orthogonal to the orientation of the tip of each microneedle 12.

In the first state S1, each microneedle 12 does not rise from the main surface 11a of the sheet body 11 and lies along the main surface 11a. In other words, the respective tips of the microneedles 12 are oriented to the second end 14 (second side 92). It can also be said that the angle formed by each microneedle 12 and the sheet body 11 (main surface 11a) is 0 degrees or about 0 degrees.

The second state S2 arises in response to the sheet body 11 being bent by movement of the first end 13 of the sheet body 11. For example, the second state S2 arises in response to the sheet body 11 being bent by an acute angle. In the second state S2, at least one microneedle 12 positioned in a bent portion of the sheet body 11 is raised from the main surface 11a. In the present disclosure, such a bent portion is called "first portion" for convenience. The angle formed between the microneedle 12 positioned in the first portion of the microneedle sheet 10 and the main surface 11a changes in response to the first portion being bent. Specifically, the angle increases from 0 degrees or about 0 degrees to a larger value (e.g., any value between 10 and 90 degrees). The raised microneedle 12 is applied to skin. The expression "the microneedle is applied to skin" in the present disclosure means that at least the tip of the microneedle is brought into contact with a surface of the skin. This expression can further mean that a fine scratch is made on the skin by the microneedle.

In response to the bent first portion returning to the original straight state, the angle formed between the microneedle 12 positioned in the first portion and the main surface 11a decreases. In other words, the microneedle sheet 10 returns from the second state S2 to the first state S1. This angle may decrease to 0 degrees or about 0 degrees again, or does not have to return to 0 degrees or about 0 degrees.

In the present embodiment, each microneedle 12 has a triangular shape. However, the shape of the microneedle is not limited to a particular one. Although the size and orientation of the microneedles 12 are uniform in the example of FIG. 1, at least one of the size and orientation does not have to be uniform. In a case in which each microneedle 12 has a triangular shape, the lower limit of the angle of its tip may be 10 degrees or 20 degrees, for example, and the upper limit of the angle thereof may be 150 degrees or 120 degrees, for example. The distribution of the microneedles 12 in the microneedle sheet 10 may be uniform or does not have to be uniform. For example, in a case in which the microneedle sheet 10 is viewed along the longitudinal direction, a plurality of microneedles 12 may be formed on the sheet body 11 such that areas containing one or more microneedles 12 (needle areas) and areas containing no microneedles 12 (clearance areas) are alternately arranged.

The dimensions of the microneedle sheet 10 are not limited to particular ones. Specifically, the lower limit of thickness may be 5 micrometers, 10 micrometers, 20 micrometers, 30 micrometers, 40 micrometers, or 50 micrometers, and the upper limit of the thickness may be 1000 micrometers, 500 micrometers, 300 micrometers, 200 micrometers, 150 micrometers, 100 micrometers, 90 micrometers, 80 micrometers, 70 micrometers, 60 micrometers, or 50 micrometers. This thickness is the thickness of the sheet body 11, and is also the thickness of the microneedle 12. Thus, the lower limit of the thickness of the microneedle sheet 10 may be determined in consideration of the strength of the microneedle 12 to be applied to the skin. The upper limit of the thickness may be determined in consideration of the flexibility of the sheet body 11, for example. The lower limit of length of the microneedle sheet 10 may be 0.1 centimeter or 1 centimeter, and the upper limit of the length may be 50 centimeters or 20 centimeters. The lower limit of width of the microneedle sheet 10 may be 0.1 centimeter or 1 centimeter, and the upper limit of the width may be 60 centimeters or 30 centimeters. The lower limits of length and width of the microneedle sheet 10 may be determined in consideration of the amount of an active ingredient to be administered. The upper limits of length and width thereof may be determined in consideration of the size of a living body.

The lower limit of height of the microneedle 12 may be 10 micrometers, 100 micrometers, or 200 micrometers, and the upper limit thereof may be 10000 micrometers, 1000 micrometers, or 500 micrometers. The height of the microneedle is the length from the base to the tip. The lower limit of density of the microneedles 12 may be 0.05 needle/cm² or 1 needle/cm², and the upper limit of the density may be 10000 needles/cm² or 5000 needles/cm². The lower limit of the density may be calculated based on the number of the microneedles 12 and the area that enable administration of 1 milligram of an active ingredient. The upper limit of the density may be set based on a limit value determined in consideration of the shape of the microneedle 12.

The materials of the microneedle sheet 10 and the microneedles 12 are not limited to particular ones. In one example, the materials are selected so that the microneedle sheet 10 can have flexibility that enables the microneedle sheet 10 to be folded back or follow a surface profile of skin. For example, the microneedle sheet 10 and the microneedles 12 may be made from any of stainless steel, polyethylene terephthalate (PET), polyvinyl alcohol (PVA), poly(lactic-co-glycolic acid) (PLGA), polylactic acid (PLA), pullulan, water-soluble polymers, other metals, other resins, biodegradable materials, ceramics, and bioabsorbable materials. Alternatively, the microneedle sheet 10 and the microneedles 12 may be made from these materials in combination.

In the present disclosure, "materials of the microneedle sheet and the microneedles" refer to substances for a manufacturer to intentionally use to produce the microneedle sheet and the microneedles. In manufacturing processes of the microneedle sheet and the microneedles, there may be a situation in which a substance (e.g., a slight amount of impurities) that has not been selected as a material is unintentionally mixed therein, or such an unintentional substance cannot be completely removed. The microneedle sheet and the microneedles according to the present disclosure also cover a microneedle sheet and microneedles eventually including a substance that the manufacturer does not intend to use in addition to materials that the manufacturer does intend to use.

The microneedles 12 can be formed by etching. In a case in which the sheet is metal, the microneedles 12 can be formed by partially dissolving the sheet with a chemical solution. In a case in which the sheet is nonmetal, the microneedles 12 can be formed by partially cutting the sheet with a laser. Alternatively, by a two-stage process of making a first cut in the sheet of metal or nonmetal and then making a second cut connecting to the first cut in the sheet, the tip of each microneedle 12 may be formed. In these cases, a void is formed around the microneedle 12. As a matter of course, the microneedles 12 may be formed by a method other than laser processing and etching. In any case, the microneedles 12 do not have to be raised from the main surface 11a of the sheet in advance, and thus the microneedle sheet 10 can be produced easily at low cost.

A method for preparing an active ingredient to be applied to skin is not limited to a particular one. For example, the microneedle sheet 10 itself (more specifically, the microneedles 12 themselves) may contain the active ingredient in advance. Alternatively, the microneedle sheet 10 itself may be coated with the active ingredient in advance. Alternatively, the active ingredient may be rubbed onto skin before the microneedles 12 is applied to the skin. Alternatively, after the microneedles 12 are applied to skin, the active ingredient may be rubbed onto the skin, or a patch containing the active ingredient may be stuck to the skin. In a case in which the microneedle sheet 10 is coated with the active ingredient in advance, coating liquid having a predetermined viscosity is applied in preferably uniform thickness to the entire sheet, for example. Such uniform application can be easily performed because the tips of the microneedles 12 are oriented toward one end of the sheet body 11 (i.e., because the microneedles 12 lie along the main surface 11a). The coating may be performed by using a principle of screen printing, or may be performed by using another method. The microneedle sheet 10 itself can contain the active ingredient in a case in which, for example, a biodegradable sheet or a sheet made with water-soluble polymer is used.

The microneedle sheet 10 may be provided in such a form that the main surface 11a is protected by a release liner. Examples of material of the release liner include plastics such as PET. However, the material is not limited to a particular one, and metal and another type of resin may be used, for example. In a case in which the microneedle sheet 10 is protected by the release liner, the microneedle sheet 10 is fixed or temporarily attached to one side of the release liner with a tape or adhesive, for example.

The respective microneedles 12 are in the state of lying along the main surface 11a of the sheet body 11 before being bent. Thus, in a stage before the microneedle sheet 10 is actually used, there is no need to worry that the microneedles 12 may come into contact with or be caught in another object (e.g., skin or clothes of a user). Consequently, safety of the microneedles 12 when being handled can be obtained. For example, the user can safely perform storage and transfer of the microneedle sheet 10, and preparation thereof immediately before using it.

### [Method for Using Microneedle Sheet, which is not part of the invention]

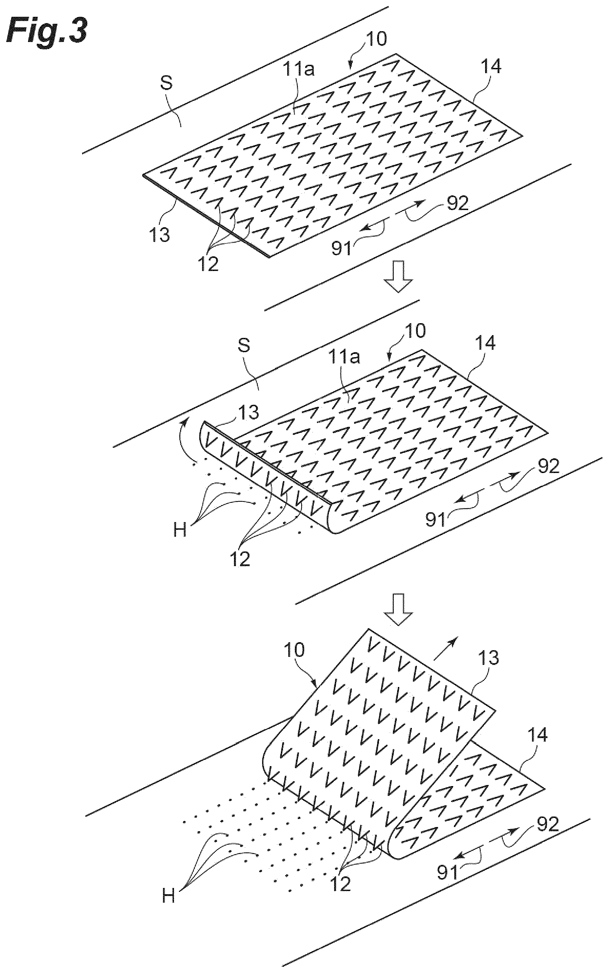

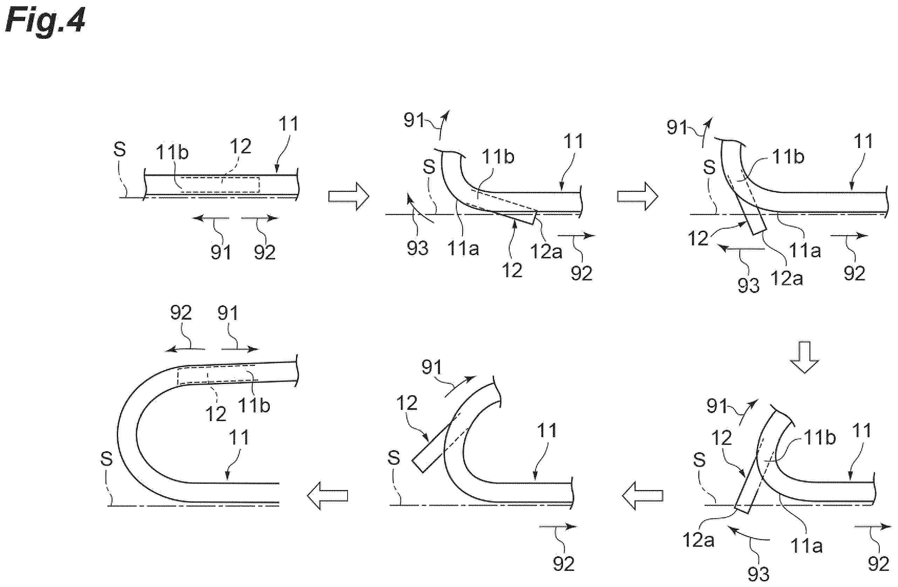

Referring to FIG. 3 and FIG. 4, the following describes a method for using the microneedle sheet 10. FIG. 3 is a diagram illustrating one example of the method for using the microneedle sheet 10. FIG. 4 is a diagram illustrating one example of rising of the microneedles 12.

In these examples, to begin with, the microneedle sheet 10 that is not bent is placed on skin S. In this stage, a side surface of each microneedle 12 is in contact with the skin S, but the tip of the microneedle 12 is not yet in contact with the skin S. Subsequently, the first end 13 of the sheet body 11 is pulled in a direction away from the skin S, whereby the first portion of the sheet body 11 is bent from the first side 91. The expression "the first portion of the sheet body is bent from the first side" means that the first portion is bent from a side on which the base of the microneedle 12 is positioned (i.e., the first side) earlier than a side on which the tip of the microneedle 12 is positioned (i.e., the second side). In one example, the first portion of the sheet body 11 is bent by an acute angle from the first side 91. The expression "the first portion of the sheet body is bent by an acute angle" means that the first portion is bent such that the angle formed between a first side portion of the sheet body positioned closer to the first end 13 than the first portion and a second side portion of the sheet body positioned closer to the second end 14 than the first portion becomes less than 90 degrees. For example, the first portion may be bent such that the angle becomes 0 degrees or about 0 degrees, i.e., such that the sheet body 11 is turned around (in other words, such that the sheet body 11 is bent by 180 degrees).

By bending the first portion of the sheet body 11, microneedles 12 positioned in the first portion is raised from the main surface 11a, and the raised microneedles 12 are applied to the skin S. By applying the raised microneedles 12 to the skin S, fine scratches H like holes are made on the skin S. The radius of curvature indicating a manner in which the first portion is bent is not limited to a particular one, and may be set based on any requirements in consideration of various elements such as the thicknesses of the sheet body 11 and each microneedle 12 and the height of the microneedle 12. For example, the radius of curvature may be any value between 250 micrometers to 1000 micrometers.

Referring to FIG. 4, the following describes movement of a microneedle 12 with respect to the skin S more specifically. In a stage in which the first portion 11b of the sheet body 11 is bent, the angle formed between the microneedle 12 and the main surface 11a increases, which means that the microneedle 12 is raised from the main surface 11a. In this stage, a tip 12a of the raised microneedle 12 is exposed from the main surface 11a, and the exposed tip 12a comes into contact with the skin S. As the first end 13 is moved, the tip 12a shaves the skin S while advancing on the skin S along a direction 93 from the second side 92 toward the first side 91. In a stage in which the first end 13 is further moved and the first portion 11b becomes straight again, the angle formed between the raised microneedle 12 and the main surface 11a decreases, and this angle finally becomes 0 degrees or about 0 degrees. In other words, this microneedle 12 lies along the main surface 11a again.

In a case in which a plurality of microneedles 12 are aligned in one row along the width direction (second direction) in the first portion 11b, two or more microneedles 12 are simultaneously raised in the one row by bending the first portion 11b along the width direction (second direction). This means that any first microneedle selected from the microneedles 12 in the row and a second microneedle positioned adjacently to the first microneedle are simultaneously raised.

In one example, the first end of the sheet body 11 is further pulled, whereby the sheet body 11 is gradually peeled from the skin S. During this peeling, individual first portions 11b of the sheet body 11 are bent, whereby microneedles 12 positioned in the first portions 11b are raised from the main surface. The tips of the raised microneedles 12 then come into contact with the skin S and advance on the skin S along the direction 93, whereby the raised microneedles 12 are applied to the skin S. Consequently, a plurality of fine scratches H like holes are made on the skin S by the microneedles 12.

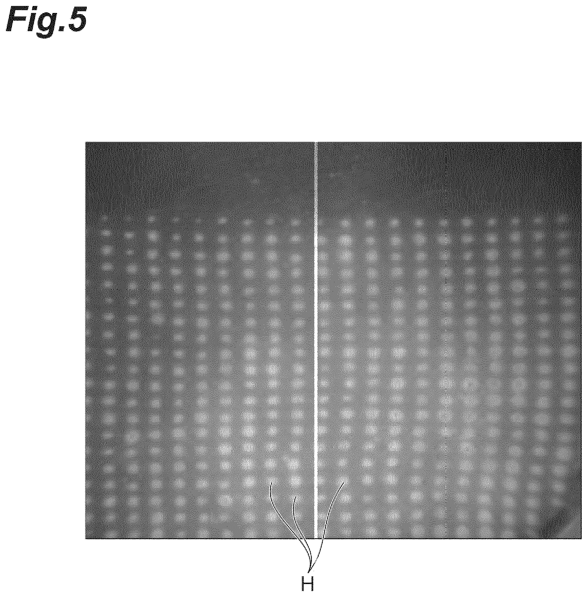

FIG. 5 is a photograph illustrating a result of an in-vitro test using human skin for checking application of the microneedle sheet 10. In this test, a microneedle sheet made from PET was used, and the length and the tip angle of each microneedle were set to 500 micrometers and 45 degrees, respectively. The radius of curvature when the microneedle sheet was bent was 300 micrometers. In this test, the sheet body was bent at the first portion by 180 degrees, whereby the microneedle was raised from the main surface of the sheet body. The human skin to which a plurality of the microneedles had been applied was dyed with rhodamine, and a picture of the human skin was taken to check formed scratches H. As is apparent from FIG. 5, the microneedles can be effectively applied to the skin by the method for using the microneedle sheet according to the present disclosure.

### [Structure of Applicator]

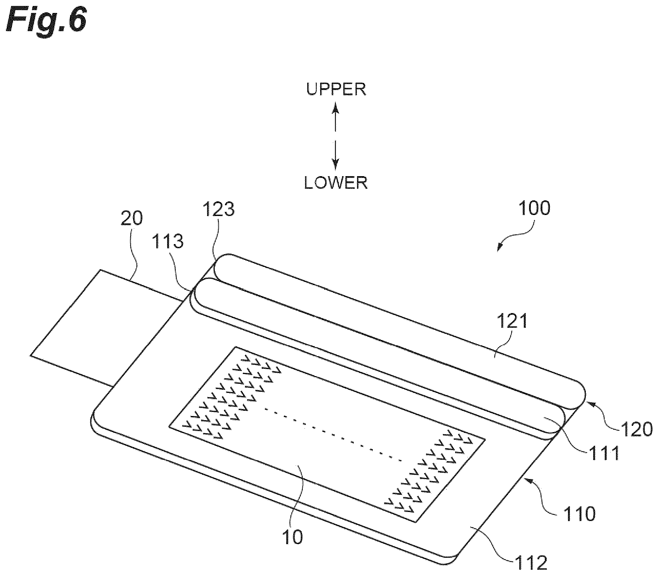

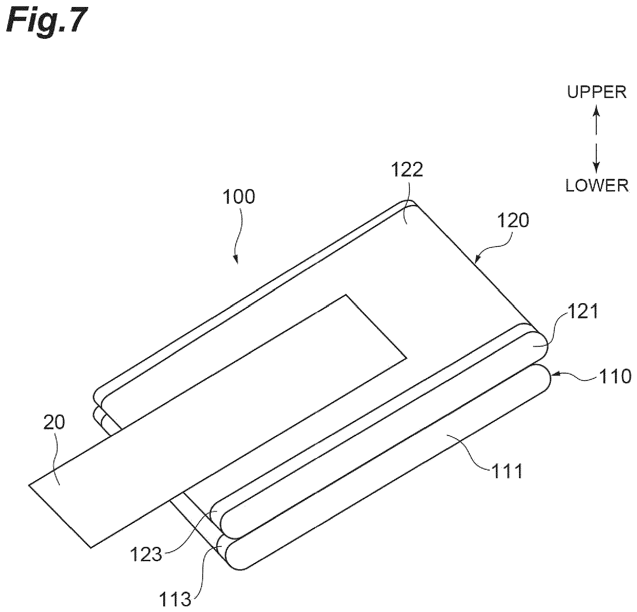

Referring to FIG. 6 and FIG. 7, the following describes one example of an applicator. FIG. 6 is a perspective view of an applicator 100 according to the embodiment when viewed from below, and FIG. 7 is a perspective view of the applicator 100 when viewed from above.

The applicator 100 is an auxiliary tool for applying the microneedle sheet 10 and a patch 20 to skin. More specifically, the applicator 100 makes fine scratches on the skin by the microneedle sheet 10, and applies the patch 20 on that spot. Consequently, an active ingredient contained in the patch 20 is transdermally administered.

The patch 20 is a pharmaceutical patch used as a gel patch, a tape patch, an impregnant (e.g., adhesive plaster), and a film patch, for example. In one example, the patch 20 is obtained by forming an adhesive layer on at least part or substantially the whole of one surface of a sheet-like backing. The backing may be formed with a woven fabric, a knitted fabric, an unwoven fabric, unwoven paper, or a film, for example, and may have stretchability. In one example, a material is selected so that the patch 20 can have flexibility for the patch 20 to be able to be folded back and follow a surface profile of skin. The adhesive layer contains any active ingredient. The expression "the adhesive layer contains an active ingredient" means a concept including both of a mode in which the adhesive layer contains the active ingredient therein and a mode in which the active ingredient sticks to a working surface of the adhesive layer.

The applicator 100 includes a first cartridge 110 to which the microneedle sheet 10 is attached and a second cartridge 120 to which the patch 20 is attached. The second cartridge 120 is stacked on the first cartridge 110. A method for coupling the first cartridge 110 and the second cartridge 120 is not limited to a particular one, and any structure or a method therefor may be used. In the present embodiment, a side on which the first cartridge 110 is positioned is defined as a lower side of the applicator 100, and a side on which the second cartridge 120 is positioned is defined as an upper side of the applicator 100.

The first cartridge 110 includes a first support plate 111 having a planar shape, a first film 112 attached to this first support plate 111 and having an annular shape, and a microneedle sheet 10 fixed to the first film 112. Both ends of the first support plate 111 covered by the first film 112 are rounded, and one end thereof serves as a bending portion 113 configured to bend the microneedle sheet 10 for applying the microneedle sheet 10 to skin. The first film 112 can be rotated around the first support plate 111. The microneedle sheet 10 is fixed to a portion of the first film 112 positioned on the lower surface side of the first support plate 111 such that bases of the respective microneedles 12 are oriented toward the bending portion 113.

The second cartridge 120 includes a second support plate 121 having a planar shape, a second film 122 attached to the second support plate 121 and having an annular shape, and the patch 20 attached to this second film 122. Both ends of the second support plate 121 covered by the second film 122 are rounded, and one end thereof serves as a feed end 123 configured to feed the patch 20 toward the skin to which the microneedles 12 have been applied. The feed end 123 is positioned above the bending portion 113. The second film 122 can be rotated around the second support plate 121. Part of the patch 20 is temporarily attached to a portion of the second film 122 positioned on the upper surface side of the second support plate 121, and the remaining part of the patch 20 protrudes outward from the feed end 123 without being stuck to the second film 122.

Material for forming the applicator 100 is not limited to a particular one. For example, material of the support plate may be plastic such as acrylic, may be metal, or may be another type of resin, for example, and these may be used in combination. Material of the film may be PET. In order to easily peel off the patch 20 from the second film 122, mold release treatment with silicone or fluorinated polyolefin, for example, may be provided to the second film 122.

The dimensions of the applicator 100 may be determined based on any requirements. For example, the width of the applicator 100 may be determined based on the widths of the microneedle sheet 10 and the patch 20. The height and the entire length (length along the longitudinal direction) of the applicator 100 may be determined in consideration of various elements such as operability of the applicator 100 and the area of the microneedle sheet 10 to be applied to skin. The shape or the radius of curvature of the bending portion 113 may be designed based on any policy in consideration of application of the microneedles 12 to the skin.

### [Method for Using Applicator]

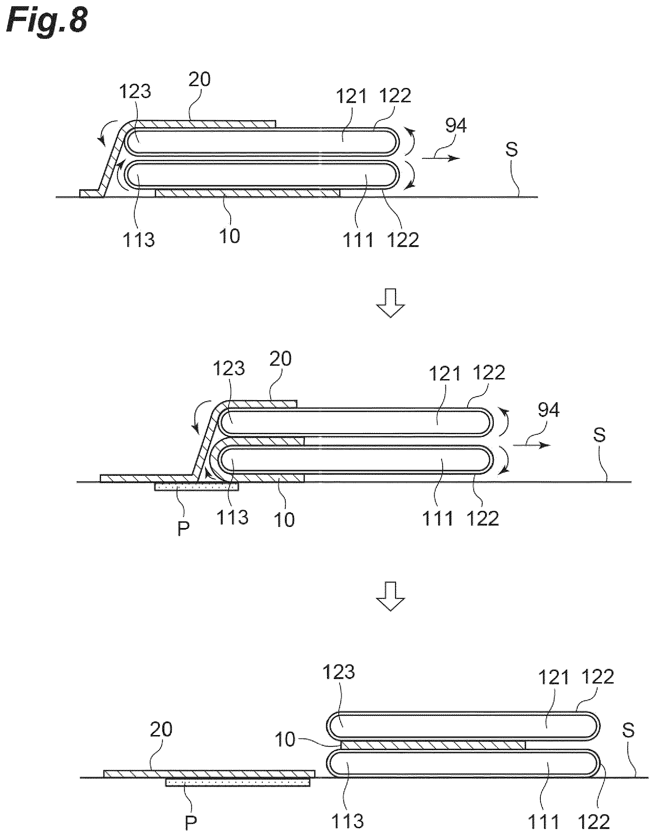

FIG. 8 is a diagram illustrating one example of a method for using the applicator 100 , which is not part of the invention, and, more specifically, is a diagram illustrating one example of a method for using the applicator 100 , which is not part of the invention, to apply the microneedle sheet 10 and the patch 20 to skin.

The user places the applicator 100 upon skin S such that the first cartridge 110 is positioned below the second cartridge 120, and sticks an end portion of the patch 20 protruding from the feed end 123 onto the skin S. In this stage, side surfaces of the respective microneedles 12 are in contact with the skin S, but the tips of the microneedles 12 are not yet in contact with the skin S.

Subsequently, the user moves the applicator 100 on the skin S along a moving direction 94 that is a direction opposite to a side on which the bending portion 113 and the feed end 123 are positioned. By this operation, in the first cartridge 110, the first film 112 is rotated so as to move upward from below at the bending portion 113 (in FIG. 8, this rotation is indicated as a clockwise motion). By this rotation, the bending portion 113 is brought closer to the microneedle sheet 10. Meanwhile, in the second cartridge 120, the second film 122 is rotated so as to move downward from above at the feed end 123 (in FIG. 8, this rotation is indicated as a counterclockwise motion). By this rotation, the part of the patch 20 temporarily attached to the second film 122 advances toward the feed end 123.

When a first portion of the sheet body 11 is positioned on the bending portion 113, the first portion is bent by an acute angle (more specifically, the first portion is bent such that the sheet body 11 is folded back), whereby microneedles 12 positioned in the first portion are raised from the main surface 11a. The raised microneedles 12 are applied to the skin S as illustrated in FIG. 4. When the user continues to move the applicator 100, the patch 20 that has been fed from the feed end 123 is stuck to an area P to which the microneedles 12 have been applied. The user moves the applicator 100 along the moving direction 94 until the whole of the microneedle sheet 10 is moved to the upper side of the first support plate 111 and the whole of the patch 20 is fed onto the skin S from the feed end 123. Consequently, the patch 20 is stuck to the area P in which a plurality of fine scratches have been made by the microneedles 12.

The user can perform, by a single action of moving the applicator 100 along the moving direction 94, a series of operations of applying the microneedle sheet 10 to skin first and then applying the patch 20 to the skin. By moving the applicator 100 for a desired distance, the user can adjust the application areas of the microneedle sheet 10 and the patch 20 and administer the active ingredient in a desired amount. By using the applicator 100, anyone can apply the microneedle sheet 10 and the patch 20 to skin by the same method. This means that variations in application of the microneedle sheet 10 and the patch 20 to the skin can be reduced.

### [Effects]

As described above, a microneedle sheet according to one aspect of the present disclosure includes: a sheet body having a main surface; and at least one microneedle formed on the sheet body and lying along the main surface. Each of the at least one microneedle has a base positioned on a first side of the sheet body and a tip oriented toward a second side of the sheet body that is opposite to the first side. A first portion of the sheet body is bent from the first side, whereby the microneedle positioned in the first portion is raised from the main surface and the raised microneedle is applied to skin.

In this aspect, the microneedle is in the state of lying along the main surface of the sheet body until the sheet body is bent. This means that the tip of the microneedle does not protrude from the main surface until the microneedle is applied to the skin. Thus, unless the microneedle sheet is applied to the skin, there is no need to worry that the microneedle may come into contact with or be caught in another object. Consequently, safety of the microneedle when being handled can be obtained.

Furthermore, the microneedle is raised from the main surface of the sheet body and is applied to the skin, and thus the microneedle can be applied to the skin without exerting an impact on the microneedle sheet. This enables administration of the active ingredient without giving a sense of fear to a subject of the administration.

In the microneedle sheet according to another aspect, the first portion of the sheet body may be bent by an acute angle. By bending the sheet body in this manner, the microneedle can be more reliably raised from the main surface of the sheet body.

In the microneedle sheet according to another aspect, the tip of the raised microneedle may be brought into contact with the skin, and the tip being in contact with the skin may be caused to advance on the skin along a direction from the second side toward the first side, whereby the raised microneedle may be applied to the skin. By moving the microneedle in this manner, a fine scratch is made on the skin, and thus the active ingredient can be more effectively administered into a living body through the opening of the scratch.

In the microneedle sheet according to another aspect, after the raised microneedle has been applied to the skin, the microneedle may lie along the main surface again. Because the microneedle is raised from the main surface of the sheet body only in a stage of being applied to the skin, the safety of the microneedle when being handled can be obtained not only before use of the microneedle sheet but also after the use thereof.

In the microneedle sheet according to another aspect, before the first portion is bent, a side surface of the microneedle positioned in the first portion may be in contact with the skin. By positioning the first portion in this manner, the microneedle can be more reliably raised from the main surface of the sheet body when the first portion is bent.

In the microneedle sheet according to another aspect, the sheet body may have a belt shape, the at least one microneedle may be a plurality of microneedles, and the microneedles may be formed so as to be aligned in one row in each of a first direction of the sheet body extending from the first side to the second side and a second direction orthogonal to the first direction. By arranging, in the belt-shaped sheet body, the microneedles regularly in this manner, the microneedles can be effectively applied to the skin.

In the microneedle sheet according to another aspect, the first portion may be bent along the second direction, whereby two or more microneedles positioned in the first portion may be simultaneously raised. By bending the first portion in this manner, the microneedles can be effectively applied to the skin.

An applicator according to one aspect of the present disclosure includes: a microneedle sheet; and a bending portion configured to bend the microneedle sheet. The microneedle sheet includes a sheet body having a main surface and at least one microneedle formed on the sheet body and lying along the main surface. Each of the at least one microneedle has a base positioned on a first side of the sheet body and a tip oriented toward a second side of the sheet body that is opposite to the first side. In response to the bending portion bending a first portion of the sheet body from the first side, the microneedle positioned in the first portion is raised from the main surface and the raised microneedle is applied to skin.

In this aspect, the microneedle is in the state of lying along the main surface of the sheet body until the sheet body is bent. This means that the tip of the microneedle does not protrude from the main surface until the microneedle is applied to the skin. Thus, unless the microneedle sheet is applied to the skin, there is no need to worry that the microneedle may come into contact with or be caught in another object. Consequently, safety of the microneedle when being handled can be obtained.

Furthermore, the microneedle is raised from the main surface of the sheet body and is applied to the skin, and thus the microneedle can be applied to the skin without exerting an impact on the microneedle sheet. This enables administration of the active ingredient without giving a sense of fear to a subject of the administration.

Furthermore, by using the applicator, anyone can apply the microneedle sheet to skin by the same method, and thus variations in application of the microneedle sheet to the skin can be reduced.

The applicator according to another aspect may include: a first cartridge including the microneedle sheet and the bending portion; and a second cartridge configured to feed a patch to the skin to which the microneedle has been applied. In this case, the user can easily perform, with the applicator, a series of operations of applying the microneedle sheet to the skin first and then applying the patch to the skin.

### [Modifications]

The present disclosure has been described above in detail based on the embodiment.

In the embodiments above, the longitudinal direction of the belt-shaped microneedle sheet 10 is the first direction and the width direction thereof is the second direction. However, the relation between the shape of the belt-shaped microneedle sheet and the first and second directions is not limited to this. For example, the width direction of the belt-shaped microneedle sheet may be the first direction, and the longitudinal direction thereof may be the second direction. The shape of the microneedle sheet is not limited to the belt shape. For example, the shape thereof may be a rectangle having a length and a width that are substantially the same, may be a circle or an ellipse, may be another polygon such as an octagon, or may be a more complicated shape.

The microneedle sheet may be used with the applicator, or may be applied to skin without using the applicator.

The microneedle sheet can be used in combination with another transdermal absorption promoting technology including at least one of electricity (iontophoresis), pressure, a magnetic field, and ultrasound (sonophoresis). In other words, in the microneedle sheet according to another aspect, the microneedle sheet may be usable in combination with another transdermal absorption promoting technology, and the other transdermal absorption promoting technology may include at least one of electricity, pressure, a magnetic field, and ultrasound. By using the microneedle sheet in combination with these other technologies, the amount of the active ingredient to be administered can be further increased.

In the embodiments above, before the first portion of the sheet body 11 is bent, the microneedle sheet 10 (sheet body 11) is placed on skin. In other words, before the first portion of the sheet body 11 is bent, side surfaces of the respective microneedles 12 are in contact with the skin. However, if microneedles can be raised by bending the microneedle sheet and the raised microneedles can be applied to the skin, the microneedle sheet (sheet body) does not necessarily have to be placed on the skin in advance.

In the embodiments above, the applicator 100 includes the first cartridge 110 and the second cartridge 120. However, the applicator does not have to include a configuration for applying a sheet member other than the microneedle sheet to skin. For example, the above-described applicator 100 may be modified such that the second cartridge 120 is omitted.

As long as the microneedles can be raised by bending the microneedle sheet, the shape and the structure of the applicator are not limited to particular ones. For example, the applicator may have a shape like a single straight bar. Alternatively, the applicator may have any mechanical, electrical, or electronical structure or control means.

In the embodiments above, the bending portion 113 is brought closer to the microneedle sheet 10 by operation of the applicator 100, whereby the first portion of the sheet body 11 is positioned on the bending portion 113. However, the mechanism for positioning the microneedle sheet on the bending portion of the applicator is not limited to this. For example, a configuration to bring the microneedle sheet closer to the bending portion may be used.

In the embodiments above, the applicator 100 has a function of applying the microneedle sheet 10 and the patch 20 to skin. However, the sheet member used with the microneedle sheet is not limited to the patch, and a sheet member in any other type may be used with the microneedle sheet.

### Reference Signs List

10 ... microneedle sheet, 11 ... sheet body, 11a ... main surface, 11b ... first portion, 12 ... microneedle, 12a ... tip of microneedle, 13 ... first end, 14 ... second end, 20 ... patch, 91 ... first side, 92 ... second side, 100 ... applicator, 110 ... first cartridge, 111 ... first support plate, 112 ... first film, 113 ... bending portion, 120 ... second cartridge, 121 ... second support plate, 122 ... second film, 123 ... feed end, S ... skin, H ... scratch

## Claims

1. A microneedle sheet (10) comprising:
a sheet body (11) having a main surface (11a); and
a plurality of microneedles (12) formed on the sheet body (11) and lying along the main surface (11a), **characterized in that**:
each of the plurality of microneedles (12) has a base positioned on a first side (91) of the sheet body (11) and a tip (12a) oriented toward a second side (92) of the sheet body (11) that is opposite to the first side(91); and
by the microneedle sheet (10) that is not bent being placed on skin (S), a side surface of each of the plurality of microneedles (12) is in contact with the skin (S), without the tip (12a) of each of the plurality of microneedles (12) being in contact with the skin (S);
a first end (13) of the sheet body (11) of the microneedle sheet (10) placed on the skin is pulled in a direction away from the skin (S) and a first portion (11b) of the sheet body (11) is bent from the first side (91) by an acute angle, whereby the microneedle (12) positioned in the first portion (11b) is raised from the main surface (11a); and
the tip (12a) of the raised microneedle (12) is exposed from the main surface (11a) and the exposed tip (12a) shaves the skin (S) while advancing on the skin (S) along a direction (93) from the second side (92) toward the first side (91), whereby the raised microneedle (12) is applied to the skin (S).

2. The microneedle sheet (10) according to claim 1, **characterized in that**, after the raised microneedle (12) has been applied to the skin (S), the microneedle (12) lies along the main surface (11a) again.

3. The microneedle sheet (10) according to claim 1 or 2, **characterized in that**:
the sheet body (11) has a belt shape; and
the plurality of microneedles (12) are formed so as to be aligned in one row in each of a first direction of the sheet body (11) extending from the first side (91) to the second side (92) and a second direction orthogonal to the first direction.

4. The microneedle sheet (10) according to claim 3, **characterized in that** the first portion (11b) is bent along the second direction, whereby two or more microneedles (12) positioned in the first portion (11b) are simultaneously raised.

5. The microneedle sheet (10) according to any one of claims 1 to 4, **characterized in that**:
the microneedle sheet (10) is usable in combination with another transdermal absorption promoting technology; and
the other transdermal absorption promoting technology includes at least one of electricity, pressure, a magnetic field, and ultrasound.

6. An applicator (100) comprising:
the microneedle sheet (10) according to any one of claims 1 to 5; and
a bending portion (113) configured to bend the microneedle sheet (10), wherein the bending portion (113) bends a first portion (11b) of the sheet body (11) from the first side (91).

7. The applicator (100) according to claim 6, further comprising:
a first cartridge (110) including the microneedle sheet (10) and the bending portion (113); and
a second cartridge (120) configured to feed a patch (20) to the skin (S) to which the microneedle (12) has been applied.

## Patentansprüche

1. Ein Mikronadelblatt (10), umfassend:
einen Blattkörper (11) mit einer Hauptfläche (11a); und
eine Vielzahl von Mikronadeln (12), die auf dem Blattkörper (11) ausgebildet sind und entlang der Hauptfläche (11a) liegen, **dadurch gekennzeichnet, dass**:
jede der mehreren Mikronadeln (12) eine an einer ersten Seite (91) des Blattkörpers (11) angeordnete Basis und eine Spitze (12a) aufweist, die zu einer zweiten Seite (92) des Blattkörpers (11) hin ausgerichtet ist, die der ersten Seite (91) gegenüberliegt; und
wenn das nicht gebogene Mikronadelblatt (10) auf die Haut (S) aufgelegt wird, eine Seitenfläche jeder der mehreren Mikronadeln (12) mit der Haut (S) in Kontakt steht, ohne dass die Spitze (12a) jeder der mehreren Mikronadeln (12) mit der Haut (S) in Kontakt steht;
ein erstes Ende (13) des Blattkörpers (11) des auf die Haut aufgebrachten Mikronadelblatts (10) in einer Richtung von der Haut (S) weggezogen wird und ein erster Abschnitt (11b) des Blattkörpers (11) von der ersten Seite (91) um einen spitzen Winkel gebogen wird, wodurch die Mikronadel (12), die sich im ersten Abschnitt (11b) befindet, von der Hauptfläche (11a) abgehoben wird; und
die Spitze (12a) der abgehobenen Mikronadel (12) aus der Hauptfläche (11a) freigelegt wird und die freigelegte Spitze (12a) die Haut (S) streift, während sie sich auf der Haut (S) entlang einer Richtung (93) von der zweiten Seite (92) zur ersten Seite (91) vorwärtsbewegt, wodurch die angehobene Mikronadel (12) auf die Haut (S) aufgebracht wird.

2. Das Mikronadelblatt (10) nach Anspruch 1, **dadurch gekennzeichnet, dass** die erhobene Mikronadel (12), nachdem sie auf die Haut (S) aufgebracht wurde, wieder entlang der Hauptfläche (11a) liegt.

3. Das Mikronadelblatt (10) nach Anspruch 1 oder 2, **dadurch gekennzeichnet, dass**:
der Blattkörper (11) eine Bandform aufweist; und
die Vielzahl von Mikronadeln (12) so ausgebildet ist, dass sie in einer Reihe in jeder einer ersten Richtung des Blattkörpers (11), die sich von der ersten Seite (91) zur zweiten Seite (92) erstreckt, und einer zweiten Richtung, die orthogonal zur ersten Richtung ist, ausgerichtet sind.

4. Das Mikronadelblatt (10) nach Anspruch 3, **dadurch gekennzeichnet, dass** der erste Abschnitt (11b) entlang der zweiten Richtung gebogen ist, wodurch zwei oder mehr im ersten Abschnitt (11b) angeordnete Mikronadeln (12) gleichzeitig angehoben werden.

5. Das Mikronadelblatt (10) nach einem der Ansprüche 1 bis 4, **dadurch gekennzeichnet, dass**:
das Mikronadelblatt (10) in Kombination mit einer anderen Technologie zur Förderung der transdermalen Absorption verwendbar ist; und
die andere Technologie zur Förderung der transdermalen Absorption mindestens eines der folgenden Elemente umfasst: Elektrizität, Druck, ein Magnetfeld und Ultraschall.

6. Ein Applikator (100), umfassend:
das Mikronadelblatt (10) gemäß einem der Ansprüche 1 bis 5; und
einen Biegeabschnitt (113), der so ausgebildet ist, dass er das Mikronadelblatt (10) biegt, wobei der Biegeabschnitt (113) einen ersten Abschnitt (11b) des Blattkörpers (11) von der ersten Seite (91) her biegt.

7. Der Applikator (100) nach Anspruch 6, der ferner umfasst:
eine erste Kartusche (110), die das Mikronadelblatt (10) und den Biegeabschnitt (113) enthält; und
eine zweite Kartusche (120), die so ausgebildet ist, dass sie ein Pflaster (20) an die Haut (S) zuführt, auf die die Mikronadel (12) aufgebracht wurde.

## Revendications

1. Feuille à microaiguilles (10) comprenant :
un corps de feuille (11) ayant une surface principale (11a) ; et
une pluralité de microaiguilles (12) formées sur le corps de feuille (11) et reposant le long de la surface principale (11a), **caractérisée en ce que** :
chacune de la pluralité de microaiguilles (12) présente une base positionnée sur un premier côté (91) du corps de feuille (11) et une pointe (12a) orientée vers un second côté (92) du corps de feuille (11) qui est opposé au premier côté(91) ; et
par la feuille à microaiguilles (10) qui n'est pas fléchie en étant placée sur une peau (S), une surface latérale de chacune de la pluralité de microaiguilles (12) se trouve en contact avec la peau (S), sans la pointe (12a) de chacune de la pluralité de microaiguilles (12) se trouvant en contact avec la peau (S) ;
une première extrémité (13) du corps de feuille (11) de la feuille à microaiguilles (10) placée sur la peau est tirée dans une direction opposée depuis la peau (S) et une première portion (11b) du corps de feuille (11) est fléchie depuis le premier côté (91) selon un angle aigu, moyennant quoi la microaiguille (12) positionnée dans la première portion (11b) est élevée depuis la surface principale (11a) ; et
la pointe (12a) de la microaiguille (12) élevée est exposée depuis la surface principale (11a) et la pointe (12a) exposée rase la peau (S) tout en avançant sur la peau (S) le long d'une direction (93) depuis le second côté (92) vers le premier côté (91), moyennant quoi la microaiguille (12) élevée est appliquée à la peau (S).

2. Feuille à microaiguilles (10) selon la revendication 1, **caractérisée en ce que**, après que la microaiguille (12) élevée a été appliquée à la peau (S), la microaiguille (12) repose à nouveau le long de la surface principale (11a).

3. Feuille à microaiguilles (10) selon la revendication 1 ou 2, **caractérisée en ce que** :
le corps de feuille (11) présente une forme de courroie ; et
la pluralité de microaiguilles (12) sont formées afin d'être alignées en une rangée dans chacune d'une première direction du corps de feuille (11) s'étendant depuis le premier côté (91) vers le second côté (92) et une seconde direction orthogonale à la première direction.

4. Feuille à microaiguilles (10) selon la revendication 3, **caractérisée en ce que** la première portion (11b) est fléchie le long de la seconde direction, moyennant quoi deux ou plusieurs des microaiguilles (12) positionnées dans la première portion (11b) sont simultanément élevées.

5. Feuille à microaiguilles (10) selon l'une quelconque des revendications 1 à 4, **caractérisée en ce que** :
la feuille à microaiguilles (10) peut être utilisée en combinaison avec une autre technologie de promotion de l'absorption transdermique ; et
l'autre technologie de promotion de l'absorption transdermique inclut au moins l'un parmi l'électricité, la pression, un champ magnétique, et des ultrasons.

6. Applicateur (100) comprenant :
la feuille à microaiguilles (10) selon l'une quelconque des revendications 1 à 5 ; et
une portion de flexion (113) configurée pour fléchir la feuille à microaiguilles (10), dans lequel la portion de flexion (113) fléchit une première portion (11b) du corps de feuille (11) depuis le premier côté (91).

7. Applicateur (100) selon la revendication 6, comprenant en outre :
une première cartouche (110) incluant la feuille à microaiguilles (10) et la portion de flexion (113) ; et
une seconde cartouche (120) configurée pour alimenter un timbre (20) au niveau de la peau (S) auquel la microaiguille (12) a été appliquée.
